# DEMANDE DE BREVET EUROPEEN

(11) **EP 1 369 167 A1**
(43) Date de publication de la demande: **10.12.2003**
(21) Numéro de dépôt: 03291118.2
(22) Date de dépôt: 14.05.2003
(51) Int. Cl.: B01D 71/02

(54) **Procédé de preparation d'une membrane zéolithique de faible épaisseur**

(30) Priorité: 03.06.2002 FR 0206818
(71) Demandeur: Institut Français du Pétrole, 92852 Rueil-Malmaison Cedex (FR)
(72) Inventeur: Chau, Christophe, 92500 Rueil Malmaison (FR); Sicard, Mickael, 91120 Palaiseau (FR); Le Dred, Ronan, 68400 Riedisheim (FR)

(57) **Abrégé**

On décrit un procédé de préparation d'une membrane zéolithique supportée constituée d'une couche composite zéolithe/support, dont la phase zéolithique présente une cristallinité d'au moins 85 %, comprenant :
a) la formation d'un gel ou d'une solution comprenant au moins une source de silice et de l'eau, additionné d'au moins un composé organique polaire,
b) la mise en contact dudit gel ou de ladite solution avec un support poreux,
c) la cristallisation de la zéolithe à partir dudit gel ou de ladite solution ; et,
d) l'élimination d'agents résiduels.

Le rapport molaire de l'eau à la silice dans le gel ou la solution de l'étape a) est compris entre 27:1 et 35:1. La durée de la cristallisation de l'étape c) est d'au moins 25 heures.

Ledit procédé est particulièrement adapté pour la préparation de membranes zéolithiques dont la phase zéolithique est de type structural MFI.

## Description

La présente invention se rapporte au domaine des membranes zéolithiques supportées utilisées en séparation.

Elle a plus particulièrement pour objet un procédé de préparation contrôlée d'une membrane zéolithique supportée, les membranes zéolithiques obtenues par ce procédé et leur utilisation en séparation.

Divers procédés d'élaboration de membranes zéolithiques ont déjà été décrits. Il apparaît à ce jour difficile d'obtenir de manière contrôlée et reproductible des membranes zéolithiques dont la couche contenant la zéolithe est continue et fine. La finesse et la continuité d'une telle couche sont des paramètres essentiels pour obtenir un matériau membranaire présentant des propriétés intéressantes qui puissent être mises à profit dans des procédés de séparation industriels. Notamment, il est particulièrement difficile de maîtriser la préparation des membranes zéolithiques : les procédés d'obtention font intervenir plusieurs étapes et il est souvent nécessaire de reproduire l'étape de cristallisation à plusieurs reprises, pour obtenir, à la suite d'étapes consommatrices en temps, en coûts opératoires, en produits chimiques et en énergie, une couche continue qui puisse être mise en oeuvre en séparation. Par ailleurs, la stabilité thermique et mécanique de ces membranes inorganiques est cruciale. En effet, les matériaux inorganiques, peuvent en général, être mis en oeuvre à des températures relativement élevées, par exemple plus élevées que les membranes polymères organiques qui opèrent généralement à une température inférieure à 100°C. Il est alors essentiel, pour une application industrielle et commerciale, de disposer d'une membrane qui puisse demeurer stable lors d'opérations et de mises en oeuvre à des températures élevées voire de pressions élevées. La voie hydrothermale mettant en jeu des supports poreux présente l'avantage de stabiliser les cristaux de zéolithes dans la porosité d'une matrice poreuse (alumine, inox par exemple) et également à la surface de celle-ci.

Dans la demande de brevet EP-A-0 778 075, il est décrit un procédé d'élaboration de membranes de zéolithe supportée par du verre poreux. Le brevet US-A-5,429,743 et la demande de brevet internationale WO-A-95/29751 décrivent des protocoles d'obtention de membranes composites supportées par une matrice macroporeuse inorganique. On pourra également se référer aux documents US-A-4,099,692, WO-A-93/19840, US-A-5,567,664 et WO-A-96/01683. Dans la demande de brevet internationale WO-A-00/33948, il est décrit un procédé d'obtention de membranes composites de zéolithe supportée sur des solides tubulaires éventuellement multi-canaux. L'ensemble de ces matériaux membranaires composites à base de zéolithe est formé d'une phase zéolithique déposée sur un support. Une série de brevets récents (US-A-5,871,650, US-A-5,968,366, US-A-6,090,289, US-A-6,074,457, WO-A-00/53297, WO-A-00/53298) décrit la préparation de membranes zéolithiques dont la phase zéolithique de type structural MFI se trouve à la surface externe d'un support poreux. Un inconvénient des membranes selon l'art antérieur est qu'elles présentent généralement des défauts de texture, une cristallinité insuffisante et/ou la présence de zone(s) amorphe(s) ce qui influe de façon négative sur les performances de la séparation moléculaire et notamment sur la sélectivité. La présence d'espaces vides entre les cristaux de zéolithes et/ou la présence de phase amorphe altère notablement la sélectivité de la séparation.

La cristallisation de la zéolithe s'effectue généralement par des traitements hydrothermaux multiples d'un mélange contenant les précurseurs de la phase zéolithique, ce qui augmente l'épaisseur effective de la couche séparatrice. Lorsque l'étape de cristallisation de la zéolithe est reproduite à plusieurs reprises, la synthèse est reproduite après retour éventuel du matériau à température ambiante, lavage et séchage dudit matériau.La répétition et la succession d'opérations identiques pour la préparation de membranes zéolithiques permettent le dépôt de couches successives et/ou la formation de cristaux de zéolithes qui comblent les espaces interparticulaires, ce qui permet l'obtention d'une couche continue pour la séparation. Ce mode de synthèse en plusieurs étapes, s'il conduit à l'obtention d'une couche continue, conduit également à l'obtention de couches épaisses de zéolithes, qui risquent de se fissurer lors de la calcination de la membrane (Vroon, Z.A.E.P., Keizer, K., Burggraaf, A.J., Verweij, H., J. *Membr. Sci.* 144 (1998) 65-76), de la mise en régime de l'unité de séparation par membrane ou de la mise en oeuvre à haute température. Par ailleurs, l'augmentation d'épaisseur peut considérablement limiter le transfert de matière à travers la membrane lors de l'opération de séparation et diminuer ainsi l'intérêt technique et économique de l'opération de séparation par membrane, dû à une réduction de productivité de ladite étape de séparation. De plus, une membrane dont la couche séparatrice est épaisse nécessite de mettre en oeuvre des surfaces importantes dudit matériau membranaire pour traiter un débit de charge de mélange à séparer, ce qui se traduit par des investissements élevés. En outre, ce mode de synthèse en plusieurs étapes nécessite une quantité importante des précurseurs de la phase zéolithique, ce qui augmente notamment le coût des matières premières et précurseurs utilisés. Il présente également l'inconvénient d'allonger la durée d'obtention du matériau membranaire et d'augmenter le coût opératoire de la séparation.

Une des difficultés liées à la préparation de membranes à base de zéolithe réside notamment dans le contrôle de la cristallisation de la zéolithe afin d'obtenir des cristaux de zéolithe bien liés au support, localisés principalement dans la porosité du support, formant ainsi une couche composite zéolithe/support continue (obtenue en obstruant les espaces vides du support par des cristaux de phase zéolithique) et fine de manière à limiter la résistance de transfert à travers le matériau membranaire. La localisation majoritaire, de préférence intégrale, de la phase zéolithique dans la porosité du support confère une très bonne résistance thermique et mécanique au matériau membranaire. Toutefois, il n'est pas exclu qu'une partie minoritaire de la phase zéolithique soit localisée à la surface externe du support.

C'est un des objets essentiels de la présente invention que de fournir un procédé permettant l'élaboration de membranes zéolithiques supportées constituées d'une couche composite zéolithe/support continue et de faible épaisseur dans lesquelles la phase zéolithique, cristallisée par un unique traitement hydrothermal, présente les caractéristiques énoncées ci-dessus. En particulier, ladite phase zéolithique, qui est active en séparation, c'est-à-dire sélective vis-à-vis des composés à séparer, est fine (de même que la couche composite zéolithe/support résultante qui présente une épaisseur inférieure à 2 µm de préférence inférieure à 1 µm et de manière très préférée inférieure à 0,5 µm) et présente en outre une cristallinité élevée. Ainsi, la quantité de solide formé de nature non-zéolithique demeure très faible et très minoritaire devant la quantité de phase zéolithique et la membrane résultante présente en conséquence des qualités séparatrices élevées, ce qui se traduit par des performances séparatrices très élevées. En particulier, la sélectivité ou pouvoir séparateur des membranes préparées selon le procédé de l'invention est très élevée voire même infinie. Par ailleurs ces membranes sont des matériaux composites, dont la couche séparatrice est formée de cristaux de zéolithe immobilisés et stabilisés dans la porosité d'un support inorganique. Ces membranes zéolithiques présentent également une très bonne intégrité structurale, c'est-à-dire une absence de défauts dans la structure de la couche composite et une absence d'espaces interparticulaires, c'est-à-dire de vides présents entre les cristaux de la zéolithe, ce qu'il est difficile d'obtenir par les procédés antérieurs en une seule étape.

Selon l'invention, le procédé de préparation d'une membrane zéolithique supportée constituée d'une couche composite zéolithe/support, dont la phase zéolithique présente une cristallinité d'au moins 85% comprend :
a) la formation d'un gel ou d'une solution comprenant au moins une source de silice et de l'eau, additionné d'au moins un composé organique polaire,
b) la mise en contact dudit gel ou de ladite solution avec un support poreux,
c) la cristallisation de la zéolithe à partir dudit gel ou de ladite solution, et
d) l'élimination d'agents résiduels,
ledit procédé étant caractérisé en ce que le rapport molaire de l'eau à la silice H₂O/SiO₂ dans ledit gel ou ladite solution dans l'étape (a) est de 27:1 à 35:1 et la durée de la cristallisation dans l'étape (c) est d'au moins 25 heures.

La cristallinité de la phase zéolithique représente la quantité relative de phase zéolithique cristallisée par rapport au solide formé à l'issue du traitement hydrothermal. Ainsi, une cristallinité d'au moins 85% signifie qu'au moins 85% en poids de solide formé à l'issue du traitement hydrothermal est de nature zéolithique, identifiable par analyse DRX, la partie non-zéolithique, sans propriété séparatrice, représentant moins de 15% en poids de solide formé à l'issue du traitement hydrothermal. Plus particulièrement, une cristallinité de 100% signifie donc l'obtention d'une membrane zéolithique présentant 100 % de quantité relative de zéolithe cristallisée et l'absence de toute phase amorphe ou solide sans propriétés de séparation. Conformément à l'invention, la phase zéolithique, de faible épaisseur, de la membrane zéolithique présente avantageusement une cristallinité d'au moins 90 %.

De préférence, le rapport molaire de l'eau à la silice H₂O/SiO₂ dans ledit gel ou ladite solution dans l'étape (a) est de 27:1 à 32:1. Avantageusement, l'étape (a) du procédé selon l'invention est réalisée en utilisant des rapports molaires de l'eau à la silice dans ledit gel ou ladite solution de 28:1 à 31:1.

La source de silice employée dans l'étape (a) du procédé selon l'invention est de préférence une silice colloïdale ou une silice précipitée. Il peut également s'agir d'ions silicates tels que le silicate de sodium, d'alcoxydes de silicium ou du tétrachlorure de silicium.

D'autres éléments peuvent également être introduits en quantité minoritaire lors de l'étape (a) du procédé selon l'invention. En particulier, l'aluminium, le bore, le gallium, le germanium, le titane et le phosphore ainsi que le mélange de ces éléments peuvent être ajoutés lors de l'étape (a).

Conformément à l'invention, le composé organique polaire additionné au gel ou à la solution comprenant au moins ladite source de silice et l'eau est de préférence un composé basique. Il s'agit avantageusement d'hydroxydes organiques, tel que l'hydroxyde de tétrapropylammonium, d'agents structurants organiques contenant des paires ioniques (ions ammonium ou phosphonium et les anions correspondants) ou des molécules neutres (aminés, alcools ou éthers tels que les éthers-couronnes et les cryptands). Le rapport molaire du composé organique polaire à la silice est compris entre 0,3:1 et 0,6:1 et de préférence entre 0,35:1 et 0,50:1. Les ions hydroxydes ou fluorures peuvent par ailleurs être utilisés pour la dissolution des précurseurs et sont introduits dans le milieu de préparation par exemple sous forme d'hydroxyde de sodium, d'hydroxydes organiques et d'acide fluorhydrique.

Le support poreux employé dans l'étape b) du procédé selon l'invention est préférentiellement constitué d'un matériau inorganique. Un support en céramique à base d'alumine et/ou de zircone et/ou d'oxyde de titane est un support approprié. D'autres matériaux, dont la nature suit, peuvent également convenir : carbone, silice, zéolithes, argiles, verre, métal (acier inoxydable, argent). L'utilisation d'un support en alumine de variété allotropique alpha ou gamma est préférée. Toutes les géométries peuvent convenir pour le support et celui-ci peut être, par exemple, tubulaire, spiralé, plan, sous forme de disque, de feuilles ou encore de fibres, notamment de fibres creuses dont le rapport surface/volume (compacité) est élevé.

Avantageusement, la durée de cristallisation de la zéolithe dans l'étape (c) est d'au moins 40 heures et de manière encore plus avantageuse elle est d'au moins 65 heures. Conformément à l'invention, la cristallisation de la zéolithe est réalisée en une seule étape, c'est-à-dire que la zéolithe est cristallisée par un unique traitement hydrothermal.

L'élimination des agents résiduels, principalement du composé organique polaire, conformément à l'étape (d) du procédé selon l'invention, est effectuée par traitement thermique réalisé entre 350 et 550°C, de préférence entre 400°C et 500°C, dans un four sous atmosphère d'air ou sous atmosphère N₂/O₂ dans des proportions variables. Après élimination de ces agents résiduels, la microporosité des membranes zéolithiques peut alors être mise en oeuvre pour une opération de séparation.

Le procédé selon l'invention est particulièrement adapté pour la préparation de membranes zéolithiques dont la phase zéolithique est de type structural MFI.

L'invention concerne aussi les membranes zéolithiques supportées obtenues par le procédé de l'invention, dans lesquelles la phase zéolithique présente une cristallinité d'au moins 85 %, de préférence d'au moins 90 %, ladite phase étant fine et localisée principalement, de préférence intégralement, dans la porosité dudit support poreux. Lesdites membranes présentent, dans la séparation n-butane/isobutane, une perméance de n-butane d'au moins 3.10⁻⁷ mol/m².s.Pa à la température de 180°C.

On rappelle que la perméance d'un gaz, exprimée en mole/m².s.Pa, est, par définition, le débit molaire de ce gaz ramené à l'unité de surface membranaire et ramené à la différence de pression partielle de ce gaz entre l'amont (où circule la charge) et l'aval (où l'on récupère le perméat). La perméance d'un gaz est donc le débit molaire de ce gaz traversant la membrane par unité de surface et de pression. La sélectivité α (appelée permsélectivité) est, dans le cas de mesures de perméation de corps purs, le rapport des perméances de ces corps purs, c'est-à-dire dans le cadre de la présente invention le rapport des perméances du n-butane et de l'isobutane.

Les membranes zéolithiques obtenues par le procédé de l'invention peuvent être utilisées pour différentes séparations. Elles sont avantageusement utilisées dans des procédés de séparation de gaz, de séparation de vapeurs ou de séparation de liquides. Ainsi, elles sont préférentiellement utilisées pour séparer
- des paraffines linéaires et branchées (n- et iso-paraffines), comme par exemple le n-butane et l'isobutane,
- des paraffines branchées entre elles (mono-branchées et di-branchées ou multi-branchées),
- des oléfines linéaires et branchées (n- et iso-oléfines),
- des paraffines et des oléfines,
- des naphtènes et des paraffines,
- des paraffines et des aromatiques,
- de l'hydrogène et des hydrocarbures ; par exemple, dans les mélanges contenant l'hydrogène et les hydrocarbures suivants, présents séparément ou simultanément, les paraffines légères comme le méthane, l'éthane, le propane ou le butane et l'isobutane ; ou encore l'hydrogène et les oléfines légères comme l'éthylène, le propylène et les isomères des butènes, l'isobutène ; ou bien encore l'hydrogène et les hydrocarbures polyinsaturés comme l'acétylène, le propyne, butyne et butadiène, ces hydrocarbures étant pris séparément avec l'hydrogène ou en mélange,
- les isomères du xylène (ortho-, méta-, para-xylènes),
- du méthane et du CO₂.

Conformément à l'invention, la qualité et les propriétés de tamisage moléculaire de la phase zéolithique sont mises à profit pour séparer des molécules dont les dimensions sont inférieures strictement d'une part et d'autre part supérieures strictement à celles des pores de la zéolithe (séparation par différentiation de taille). A titre illustratif, dans le cas de la zéolithe MFI qui présente une taille moyenne de pores de 0,55 nm (dimensions de canaux de 0.51*0.55 et 0.53*0.56 nm²), les membranes zéolithiques obtenues par le procédé de l'invention peuvent être avantageusement utilisées pour la séparation de molécules, en particulier contenant des atomes de carbone et d'hydrogène, dont les dimensions sont d'une part inférieures à 0,45 nm environ et d'autre part supérieures à 0,55 nm. Par ailleurs, les interactions entre les molécules à séparer et la phase zéolithique de la membrane peuvent également être mises à profit pour réaliser la séparation desdites molécules (séparation par adsorption).

Les exemples suivants illustrent l'invention et ne doivent en aucune manière être considérés comme limitatifs.

### Exemple 1: préparation de membranes zéolithiques (conforme à l'invention) :

Des tronçons ou éléments de tube d'alumine poreuse (de variété allotropique alpha, fournie par la société Exekia) sont utilisés comme support pour la formation de membranes zéolithiques dont la phase zéolithique est de type structural MFI. Ces éléments tubulaires sont immergés dans une solution précurseur de la zéolithe MFI. Cette solution admet pour stoechiométrie molaire 1 SiO₂ : 0,4 TPAOH : 27,0 H₂O. Elle est obtenue par mélange à température ambiante de silice type Aérosil 380® (fournie par la société Degussa) et d'une solution molaire d'hydroxyde d'ammonium tétrapropylé TPAOH (commercialisée par la société Fluka). Les éléments de support et la solution sont placés dans un autoclave horizontal à 175 °C sous rotation. Ces éléments de support, d'environ 2,5 cm de long, sont retirés après une durée de cristallisation (temps de séjour) de 72 heures dans l'autoclave. Ils sont successivement lavés à l'eau distillée et séchés à 60°C. Le composé organique polaire est éliminé par traitement thermique à 480°C.

L'analyse de phase par diffraction des rayons X sur cette membrane zéolithique obtenue après 72 heures de séjour en autoclave confirme la présence de cristaux de zéolithe MFI localisés dans la porosité du support d'alumine alpha.

On répète à trois reprises le protocole de préparation décrit ci-dessus en faisant varier pour chaque préparation la quantité d'eau introduite pour la formation de chacune des solutions précurseurs de zéolithe. On prépare ainsi 3 nouvelles membranes conformément à l'invention pour lesquelles la solution, précurseur de la zéolithe MFI, admet respectivement pour stoechiométrie molaire 1 SiO₂: 0,4 TPAOH : 28,0 H₂O, 1 SiO₂: 0,4 TPAOH : 29,7 H₂O et 1 SiO₂ : 0,4 TPAOH : 31,5 H₂O. L'analyse de phase par diffraction des rayons X sur ces 3 membranes obtenues après 72 heures de séjour en autoclave confirme la présence de cristaux de zéolithe MFI localisés dans la porosité du support d'alumine alpha.

Les résultats sont rassemblés dans le tableau 1.

**Tableau 1**

| | | | | |
|---|---|---|---|---|
| Rapport molaire H₂O/SiO₂ | 27,0 | 28,0 | 29,7 | 31,5 |
| Cristallinité (%) | 85 | 87 | 93 | 85 |

Pour des valeurs du rapport molaire H₂O/SiO₂ comprises dans l'intervalle caractérisant l'invention, les membranes zéolithiques présentent une cristallinité d'au moins 85 %. La cristallinité maximale est obtenue pour un rapport molaire H₂O/SiO₂ égal à 29,7. Les membranes zéolithiques préparées selon le procédé de l'invention sont très majoritairement formées de phase zéolithique, ce qui conduit nécessairement à des performances séparatrices très satisfaisantes (voir exemple 4).

### Exemple 2: préparation de membranes zéolithiques (non conforme à l'invention) :

Le protocole opératoire décrit dans l'exemple 1 est repris en modifiant uniquement la quantité d'eau introduite dans la solution, précurseur de la zéolithe MFI. La source de silice est l'Aérosil 380®. La durée de cristallisation est de 72 heures et l'élimination de l'hydroxyde d'ammonium tétrapropylé TPAOH est effectuée à 480°C.

On procède ainsi à 4 préparations de membranes zéolithiques pour lesquelles la solution, précurseur de la zéolithe MFI, admet respectivement pour stoechiométrie molaire 1 SiO₂ : 0,4 TPAOH : 18,3 H₂O;1 SiO₂: 0,4 TPAOH : 22,8 H₂O; 1 SiO₂ : 0,4 TPAOH : 41,2 H₂O et 1 SiO₂ : 0,4 TPAOH : 63,7 H₂O. L'analyse de phase par diffraction des rayons X sur ces 4 membranes obtenues après 72 heures de séjour en autoclave confirme la présence de cristaux de zéolithe MFI localisés dans la porosité du support d'alumine alpha.

Les résultats sont rassemblés dans le tableau 2.

**Tableau 2**

| | | | | |
|---|---|---|---|---|
| Rapport molaire H₂O/SiO₂ | 18,3 | 22,8 | 41,2 | 63,7 |
| Cristallinité (%) | 73 | 79 | 77 | 76 |

Par comparaison des résultats présentés dans les tableaux 1 et 2, il apparaît que pour des rapports molaires en dehors de l'intervalle caractérisant l'invention, les membranes zéolithiques obtenues présentent une moins bonne cristallinité que celles obtenues par un procédé dans lequel le rapport molaire H₂O/SiO₂ est compris dans l'intervalle caractérisant l'invention. Les membranes préparées selon l'exemple 2 contiennent une phase amorphe sans propriété séparatrice en bien plus grande proportion que celles préparées selon le procédé de l'invention (exemple 1).

### Exemple 3 : préparation de membranes zéolithiques (conforme à l'invention). Influence de la source de silice employée :

Le protocole de préparation est analogue à celui décrit dans l'exemple 1 mais la préparation est menée en présence d'une source de silice distincte de celle employée dans l'exemple 1. La source de silice Bindzil 40/130® sous forme colloïdale (commercialisée par la société Akzo Nobel) est utilisée pour préparer quatre membranes à partir de quatre solutions différentes dont la stoechiométrie est ISiO₂ : 0,4TPAOH : xH₂O avec x = 28,0 ; 29,7 ; 31,5 et 34,5. La durée de cristallisation est de 72 heures et l'élimination de l'hydroxyde d'ammonium tétrapropylé TPAOH est effectuée à 480°C. L'analyse de phase par diffraction des rayons X sur ces 4 membranes obtenues après 72 heures de séjour en autoclave confirme la présence de cristaux de zéolithe MFI localisés dans la porosité du support d'alumine alpha.

Les résultats sont rassemblés dans le tableau 3.

**Tableau 3**

| | | | | |
|---|---|---|---|---|
| Rapport molaire H2O/SiO2 | 28,0 | 29,7 | 31,5 | 34,5 |
| Cristallinité (%) | 91 | 99 | 94 | 90 |

Cet exemple confirme l'effet positif, déjà démontré dans l'exemple 1, du rapport molaire H₂O/SiO₂, lorsque celui-ci est compris entre 27 et 35. Les membranes zéolithiques obtenues dans cet exemple 3 présentent une cristallinité très élevée, avec une valeur maximale de 99 % pour un rapport molaire H₂O/SiO₂ égal à 29,7. La source de silice employée pour la formation de la solution, précurseur des la zéolithe MFI, n'a pas d'influence significative sur le matériau membranaire final lorsque le rapport molaire H₂O/SiO₂ est compris entre 27 et 35.

### Exemple 4 : propriétés séparatrices de membranes préparées selon le procédé de l'invention :

Deux membranes référencées A et B sont préparées à partir d'une solution de stoechiométrie H₂O/SiO₂ égale à 29,7 et à partir de tubes d'alumine alpha d'une longueur de 15 cm (surface active en séparation de 20,4 cm²). Le mode opératoire est identique à celui décrit dans l'exemple 1 : la source de silice est l'Aérosil 380®, la durée de cristallisation est de 72 heures et le traitement thermique pour éliminer le composé organique est réalisé à 480°C. La phase zéolithique est de type structural MFI.

Des mesures de séparation de gaz (perméation gazeuse) sont effectuées à 180°C sur les membranes ainsi préparées, afin d'en caractériser les propriétés et la qualité structurale, les gaz à séparer entre eux étant le n-butane et l'isobutane.

Pour ce faire, chaque membrane est insérée dans un perméateur (module de mesure de perméation) grâce à des joints de carbone qui maintiennent l'étanchéité de ce module de mesure. L'ensemble (module/membrane) est placé dans une unité de perméation gazeuse et le matériau est au préalable traité à 350 °C sous un débit d'hélium (gaz inerte), ce qui permet d'éliminer toute trace de gaz adsorbable sur la surface externe et dans la porosité interne du matériau membranaire. Durant les mesures de perméation de gaz, la membrane est soumise à une différence de pression, la pression du côté amont où circule la charge (dans cet exemple du n-butane n-C₄H₁₀ pur ou de l'isobutane pur i-C₄H₁₀) est maintenue constante à 1,5 bar (0,15 MPa) absolus et la pression du côté aval, où l'on récupère le perméat après extraction sélective d'une partie des molécules présentes dans la charge, est la pression atmosphérique. Cette différence de pression constitue la force motrice du transfert à travers la membrane. Le débit de gaz traversant la membrane est mesuré au moyen d'un débitmètre volumique. Le seuil de détection est inférieur à 0,002 mL/mn soit environ 10⁻⁶ mol/m².s de n-butane ou isobutane.

La mesure des débits de gaz traversant la membrane est effectuée avec les corps purs n-butane et isobutane. Ces molécules présentent l'avantage d'avoir des diamètres cinétiques (0,43 nm pour le n-butane et 0,49 nm pour l'isobutane) très proches des dimensions moyennes de l'ouverture des pores de la zéolithe MFI (0,55 nm). Il est à noter qu'avec le choix de ces molécules sondes que sont le n-butane et l'isobutane, cette méthode de caractérisation est considérée comme un critère très sévère et très sélectif pour caractériser des membranes inorganiques microporeuses telles que des zéolithes de type structural MFI. Elle permet dès lors de mettre en évidence la présence de toute discontinuité, de défauts, de fissures dans la couche composite zéolithe/support. A l'inverse, l'absence de défauts notables dans la membrane révèle un potentiel très élevé en séparation. En particulier, cette méthode de caractérisation utilisant le n-butane et l'isobutane est très sévère par rapport à d'autres tests de caractérisation employés dans l'art antérieur, par exemple les tests utilisant les couples N₂/SF₆, H₂/n-C₄ ou H₂/i-C₄.

Les membranes A et B présentent alors, à l'issue de mesures de caractérisation, des perméances de 3,18 et 3,05.10⁻⁷ mol/m².s.Pa de n-butane à la température de 180°C. Dans les mêmes conditions, ces matériaux sont imperméables à l'isobutane. Par suite, la sélectivité n-butane/isobutane atteint des valeurs infinies. Ces performances séparatrices sont particulièrement élevées et témoignent de l'absence totale de défauts dans la couche composite zéolithe/support ainsi que de la finesse et la continuité de ladite couche. Par comparaison, il est généralement admis dans la littérature que des membranes de type MFI présentent une bonne intégrité texturale, c'est-à-dire une absence de défauts de structure de type mésopore et macropore, lorsque la sélectivité n-butane/isobutane est supérieure à 10 (Vroon et al., J. Membr. Sci. 113 (1996) 293).

## Revendications

1. Procédé de préparation d'une membrane zéolithique supportée constituée d'une couche composite zéolithe/support, dont la phase zéolithique présente une cristallinité d'au moins 85 %, ledit procédé comprenant :
a) la formation d'un gel ou d'une solution comprenant au moins une source de silice et de l'eau, additionné d'au moins un composé organique polaire;
b) la mise en contact dudit gel ou de ladite solution avec un support poreux ;
c) la cristallisation de la zéolithe à partir dudit gel ou de ladite solution ; et
d) l'élimination d'agents résiduels,
**caractérisé en ce que**, dans l'étape (a), le rapport molaire de l'eau à la silice dans ledit gel ou ladite solution est de 27:1 à 35:1 et **en ce que**, dans l'étape (c), la durée de la cristallisation est d'au moins 25 heures.

2. Procédé selon la revendication 1 **caractérisé en ce que** dans l'étape (a) le rapport molaire de l'eau à la silice dans ledit gel ou ladite solution est compris entre 27:1 et 32:1.

3. Procédé selon l'une des revendications 1 à 2 **caractérisé en ce que** dans l'étape (a) le rapport molaire de l'eau à la silice dans ledit gel ou ladite solution est compris entre 28:1 et 31:1.

4. Procédé selon l'une des revendications 1 à 3 **caractérisé en ce que** dans l'étape (c) la durée de la cristallisation est d'au moins 65 heures.

5. Procédé selon l'une des revendications 1 à 4 **caractérisé en ce que** la phase zéolithique présente une cristallinité d'au moins 90 %.

6. Procédé selon l'une des revendications 1 à 5 **caractérisé en ce que** dans l'étape (a) le rapport molaire du composé organique polaire à la silice est compris entre 0,3:1 et 0,6:1.

7. Procédé selon l'une des revendications 1 à 6 **caractérisé en ce que** le support poreux est choisi parmi les matériaux suivants : céramique à base d'alumine et/ou de zircone et/ou d'oxyde de titane, carbone, silice, zéolithes, argiles, verre et métal.

8. Procédé selon l'une des revendications 1 à 7 **caractérisé en ce que** la phase zéolithique est de type structural MFI.

9. Membrane obtenue par un procédé selon l'une des revendications 1 à 8.

10. Membrane selon la revendication 9 **caractérisée en ce qu'**elle présente, dans la séparation n-butane/isobutane, une perméance de n-butane d'au moins 3.10⁻⁷ mol/m².s.Pa à la température de 180°C.

11. Utilisation d'une membrane selon l'une des revendications 9 à 10 dans un procédé de séparation de gaz, de séparation de vapeurs ou de séparation de liquides.

12. Utilisation d'une membrane selon l'une des revendications 9 à 10 pour la séparation des paraffines linéaires et branchées.
